(19) **Europäisches Patentamt / European Patent Office / Office européen des brevets**

(11) **EP 3 965 051 A1**

(12) # EUROPEAN PATENT APPLICATION

(43) Date of publication:
**09.03.2022 Bulletin 2022/10**

(21) Application number: **20194246.3**

(22) Date of filing: **03.09.2020**

(51) International Patent Classification (IPC):
**G06T 5/00** *(2006.01)*    **G06T 5/20** *(2006.01)*
**G06K 9/00** *(2022.01)*

(52) Cooperative Patent Classification (CPC):
**G06T 5/002; G06T 5/003; G06T 5/20;**
G06T 2207/10081; G06T 2207/20016;
G06T 2207/20081; G06T 2207/20084;
G06T 2207/30004

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **GOSHEN, Liran**
**5656 AE Eindhoven (NL)**

(74) Representative: **Philips Intellectual Property & Standards**
**High Tech Campus 52**
**5656 AG Eindhoven (NL)**

(54) **DEEP UNSUPERVISED IMAGE QUALITY ENHANCEMENT**

(57)    A training system (TS) for training a machine learning model for image quality enhancement in medical imagery. The system comprises an input interface (IN) for receiving a training input image ($\tilde{I}_{IN}$). The system (TS) comprises artificial neural network model framework (G,D) of the generative adversarial type including a generator network (G) and a discriminator (D) network. The generative network (G) processes the training input image to produce a training output image ($\tilde{I}_{OUT}$). A down-scaler (DS) of the system downscales the training input image. The discriminator attempts to discriminate between the downscaled training input image ($I'$) and training output image to produce a discrimination result. A training controller (TC) adjusts parameters of the artificial neural network model framework based on the discrimination result.

**FIG. 1**

**Description**

FIELD OF THE INVENTION

The invention relates to a training system for training a machine learning model for image quality enhancement, to trained machine learning models, to a method of training a machine learning model for image quality enhancement, to a method of image quality enhancement in medical imaging, to an imaging arrangement, to a computer program element, and to a computer readable medium.

BACKGROUND OF THE INVENTION

Enhanced image quality in medical imaging allows improved diagnostic accuracy and more appropriate management of patients.

For example, in X-ray imaging, such as CT (computed tomography) scanning, image quality (IQ) has many components and is influenced by many technical parameters.

While image quality has always been a concern for the clinical community, ensuring clinically acceptable image quality has become even more of an issue as there is more focus in the recent years on strategies to reduce radiation dose.

SUMMARY OF THE INVENTION

There may be a need for improvement in the field of image quality enhancement.

The object of the present invention is solved by the subject matter of the independent claims, with further embodiments incorporated in the dependent claims. It should be noted that the following described aspect of the invention equally applies to the trained machine learning models, to the method of training a machine learning model for image quality enhancement, to the method of image quality enhancement in medical imaging, to the imaging arrangement, to the computer program element and to the computer readable medium.

According to a first aspect of the invention there is provided a training system for training a machine learning model for image quality enhancement in medical imagery, comprising:

> an input interface for receiving a training input image ($\tilde{I}_{IN}$);
> an artificial neural network model framework of the generative adversarial type including a generator network and a discriminator network;
> the generative network to process the training input image to produce a training output image;
> a down-scaler configured to downscale the training input image, and the discriminator attempting to discriminate between the downscaled training input image and training output image to produce a discrimination result, and
> a training controller to adjust parameters of the artificial neural network model framework based on the discrimination result.

The training controller uses an objective function that represents opposed objectives for the generative network and the discriminator. The objective function may formulated as a cost/loss function or as a utility function.

In embodiments, the discriminator wherein the discriminator is configured to discriminate patch-wise. This allows for more robust training. Discrimination is based on a classification operation. In the classification, instead of processing the image as a whole, the image to be classified is first divided into subsets, the said patches, and classification is done per patch. This results in localized classification results which may then be combined to obtain the overall classification, and hence (attempted) discrimination result.

In embodiments, the generator includes a first portion having a multi-scale architecture with two processing strands, a down-scale strand and an upscale strand , the down-scale strand to downs-scale the input image to obtain a first intermediary image, and the upscale strand to upscale the intermediate image to obtain the training output image or a second intermediate image processable into the training output image.

In an alternative embodiment, the generator includes a first portion having an architecture with two processing strands, a sparsity enhancer strand and a sparsity reducer strand , the sparsity enhancer to process the input image to obtain a first intermediary image with greater sparsity than the input image, and the sparsity reducer to reduce sparsity of the intermediate image to obtain the training output image or a second intermediate image processable into the training output image.

In embodiments, the generator includes a second portion configured to process the second intermediate image into a third intermediate image , to reduce noise in the third intermediate image, and to combine the noise reduced noise image so obtained with the second intermediate image to obtain the training output image.

In embodiments, the training controller is to adjust the parameters based on any one or more of i) the third intermediate image versus a noise map computed from the input image ii) a smoothness of the second intermediate image property , iii) a dependency between a) a low-pass filtered version of the second intermediate image and b) the third intermediate image .

In another aspect there is provided a trained machine learning model obtained as the generative network of the training system , after processing one or more training input images.

In another aspect there is provided a trainable machine learning model including:

a first portion having a multi-scale architecture with two processing strands, a down-scale strand and an upscale strand , the down-scale strand to downs-scale an input image to obtain a first intermediary image, and the upscale strand to upscale the intermediate image to obtain a second intermediate image ; and
a second portion configured to process the second intermediate image into a third intermediate image , to reduce noise in the third intermediate image, and to combine the noise reduced noise image so obtained with the second intermediate image to obtain the training output image.

In another aspect there is provided a method of training a machine learning model for image quality enhancement in medical imagery, the machine learning model being a generator network of an artificial neural network model framework of the generative adversarial type, the framework further including a discriminator network, the method comprising:

receiving a training input image;
processing, by the generative network , the training input image to produce a training output image;
downscaling the training input image;
using the discriminator network to attempt discriminating between the downscaled training input image and training output image to produce a discrimination result, and
adjusting parameters of the artificial neural network model framework based on the discrimination result.

In another aspect there is provided a method of image quality enhancement in medical imaging, comprising:

receiving an input image; and
applying the trained machine learning model to the input image to obtain an output image.

In another aspect there is provided an imaging arrangement, comprising an imaging apparatus and a computing system that implements the machine learning model.

In another aspect there is provided an imaging arrangement of claim 11, wherein the imaging apparatus is any one of: i) an X-ray imaging apparatus, ii) an MR imaging apparatus, and iii) a nuclear imaging apparatus.

In embodiments, the X-ray imaging apparatus is a computed tomography scanner.

Enhanced image quality allows improved diagnostic accuracy and more appropriate management of patients. The proposed method and system enables simultaneously deblurring imagery and reducing image noise, whilst still generating enhanced images with a "classic" appearance, i.e., without looking artificial to the schooled observer. Better sharpness due to more favorable MTF (modulation transfer function) behavior may be achieved.

The approach proposed herein is preferably based on machine learning, in particular deep learning with artificial neural network models. Deep learning often requires large sets of training data to be prepared, including explicit labelling. In many cases, the labelling of such large training data sets is a challenging, tedious, time-consuming and costly task. The proposed method and systems have the attractive advantage that the model may be trained in an unsupervised manner. Labeling of training data may be infeasible or very challenging, especially in situations where one wishes to provide IQ enhancement beyond current system limitations. The proposed approach uses a training network framework, similar to generative adversarial networks ("GAN"), where the discriminator and generator networks are trained jointly, but with opposed objectives. The proposed use of the down-scaler and/or the patch-based discriminator allows improved leveraging of the GAN-setup, which results in quick, robust and well-generalized learning with good performance of the trained model, in this case the generator, in clinical practice. The down-scaler reduces image size. In general, the downscaling operation may include reducing the number of pixels/voxels. Its operation may be understood as a virtual zooming into the training input image drawn from the training data set.

In another aspect there is provided a computer program element, which, when being executed by at least one processing unit, is adapted to cause the processing unit to perform the method as per any one of the above mentioned embodiments.

In another aspect still, there is provided a computer readable medium having stored thereon the program element.

Definitions

*"user"* relates to a person, such as medical personnel or other, operating the imaging apparatus or overseeing the imaging procedure for a patient. In other words, the user is in general not the patient.

*"patient/subject"* does not exclude animals or other *"organic material"* such as bio-samples, etc. Also, inanimate objects such as an item of baggage in security checks or a product in non-destructive testing is not excluded herein as an object to be imaged, despite main reference herein to *"patient"*. The use of the term *"patient"* herein does not imply that the whole of patient is imaged. Sometimes merely a part of the object or patient is imaged, such as a particular anatomy or organ, or group of anatomies or organs of the patient.

In general, the *"machine learning"* uses a computerized arrangement that implements a machine learning ("ML") algorithm to train an ML model. The model is configured to perform a task. In an ML algorithm, task performance improves measurably after having provided the model with more (new) training data. The performance may be measured by objective tests based on test data. The performance may be defined in terms of a certain error rate to be achieved for the given test data. See for example, T. M. Mitchell, "Machine Learning", page 2, section 1.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Figure 1 shows an imaging arrangement;
Figure 2 shows an example of a modulation transfer function;
Figure 3 shows a training system for training a machine learning model for image quality enhancement;
Figure 4 shows a training system using a generative-adversarial type model architecture according to one embodiment;
Figure 5 shows a generative network according to one embodiment;
Figure 6 shows a flow chart of a method of training a machine learning model for image quality enhancement; and
Figure 7 shows a flow chart of a method of computer-implemented image quality enhancement.

DETAILED DESCRIPTION OF EMBODIMENTS

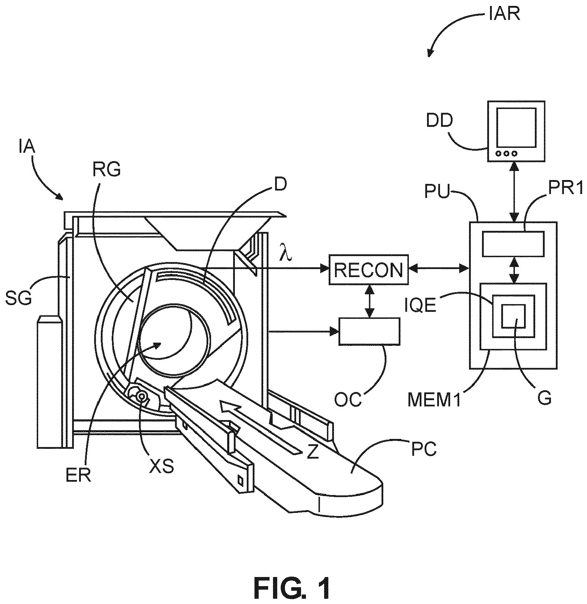

Referring first to Figure 1, this shows a schematic block diagram of an imaging arrangement IAR.

The arrangement IAR may include a medical imaging apparatus IA and a computerized image quality ("IQ") enhancer IQE, implemented on one or more data processing devices PU.

The medical imaging apparatus IA produces imagery, for example for therapy or diagnostic purposes. The imagery may be provided as sectional imagery to lay bare aspects of internal structure of physiology and/or pathophysiology of patient. The imagery may be forwarded through wired or wireless connection to the image quality enhancer IQE. The IQ enhancer IQE processes the imagery so received into quality enhanced imagery which can be displayed on a display device DD or may be stored in an image repository IR, or may otherwise be processed. The imagery may not necessarily be received by the IQ enhancer IQE directly from the imaging apparatus IA, but may be stored beforehand in an image repository. Upon user request or in an automated fashion, the image quality enhancer IQE may then access the stored imagery to then produce the image quality enhanced imagery.

Imaging operation of the imaging apparatus IA is now explained in more detail. In embodiments, the imaging apparatus is an X-ray imaging apparatus such as a CT scanner as shown in Figure 1. However, X-ray imaging modalities such as U-arm scanners are also envisaged, and so are radiography imagers.

In more detail, Figure 1 schematically illustrates a CT scanner IA. The scanner IA includes a stationary gantry SG and a rotating gantry RG. The rotating gantry is rotatably supported by the stationary gantry SG and rotates around an examination region ER and a portion of an object or subject therein about a Z-axis. A radiation source XS, such as an X-ray tube, is supported by and rotates with the rotating gantry RG around the examination region ER. The radiation source XS emits in general wideband polychromatic X-ray radiation that is optionally collimated to form generally a fan, wedge, or cone shaped X-ray radiation beam that traverses the examination region ER and hence at least a region of interest of the patient.

A radiation sensitive detector array of an X-ray detector D subtends an angular arc opposite the radiation source XS across the examination region ER. The detector array includes one or more rows of detector pixels that are arranged with respect to each other along the Z-axis and detects X-ray radiation traversing the examination region ER. The detector D provides projection (raw) data.

A reconstructor RECON reconstructs the projection raw data, generating reconstructed imagery. As will be explored in more detail below, the system IQE processes the imagery to obtain IQ enhanced imagery.

A subject support PC, such as a couch, supports the patient in the examination region ER. The subject support PC may be movable in coordination with performing the imaging operation, so as to move the patient with respect to the examination region ER for loading, scanning, and/or unloading the patient.

An operator console OC may include a human readable output device such as a display monitor, etc. and a user input device such as a keyboard, mouse, etc. The console OC further includes a processor (e.g., a central processing unit (CPU), a microprocessor, etc.) and computer readable storage medium such as physical memory. The operator console OC allows user to control the imaging operation.

The arrangement IAR may further include a processing unit PU such as workstation to image-process raw projection data acquired by the imager. The operator console OC and workstation may be arranged in the same computing system or in separate computing systems. The reconstructor RECON may run on the workstation PU.

Whilst the principles disclosed herein are described with main reference to CT or other volumetric/rotational imaging modalities such as C-arm imagers or similar, they are of equal application to projection imaging in radiography.

In more detail, during imaging operation, the patient, or at least a region of interest ("ROI") of patient, resides in the examination region ER. For example, the patient may lie on the patient couch PC arranged at least partly inside the donut shaped CT examination region ER.

The X-ray source XS is energized. X-radiation emerges from the source XS, traverses the examination region ER and the ROI/patient, and is then registered at the far end at the X-ray sensitive pixels that make up an X-ray sensitive surface of the X-ray detector D.

The impinging X-radiation causes the X-ray sensitive pixels to respond with electrical signals. The electrical signals are processed by data acquisition circuitry (not shown of the scanner IA to produce the (digital) projection raw data. The projection raw data in projection domain may be processed by the re-constructer RECON to compute, for example, cross sectional imagery of the ROI in image domain.

The re-constructer RECON is a transformer that transforms from projection domain located at the detector surface into image domain which is located in the examination region ER. The reconstructor RECON may be implemented by different types of reconstruction algorithms, such as Radon transform based algorithms, in particular filtered back-projection (FBP). Fourier-transform type algorithms are also envisaged, and so are iterative, algebraic or machine learning based reconstruction algorithms.

The reconstructed cross sectional imagery can be thought of as image values that are assigned by the re-constructer to grid points, referred to as voxels, in the 3D portion that makes up the examination region. There may be a plurality of such sectional images in different section planes of the image domain. The plurality of section images in different such planes form a 3D image volume. Location for image values in a given cross sectional image may also be referred to herein as (image) pixels.

Again, while main reference has been made above to rotational 3D X-ray imaging this is not a restriction herein as 2D radiography is also envisaged. Attenuation based imaging is envisaged, and so is phase-contrast or dark-field imaging, and other X-ray based modalities. However, nothing herein is confined to X-ray imaging and other imaging modalities are also envisaged, such as emission imaging (PET or SPECT), magnetic resonance imaging (MRI), ultrasound (US) imaging, or others still, such as (electron) microscopy, imaging of (bio)- molecules/compounds, and others. Whilst application in the medical realm is mostly envisaged herein, the proposed image quality enhancer IQE may still be used outside the medical field, such as in baggage screening or non-destructive material testing.

Turning now in more detail to the image quality enhancer IQE, this is a computerized system and is implemented as said by one or more data processing units PU. The data processing unit(s) PU may be a single general purpose computer suitably programmed. The data processing unit PU may be communicatively coupled to the imaging apparatus IA. Distributed cloud architectures are also envisaged where there are two or more processing units PU, such as servers or others, communicatively coupled to together implement the image quality enhancer IQE. A group of imagers, such as from plural imaging departments or from plural medical facilitates may be served this way.

The one or more processing units PU include one or more processors PR1 to process data by running one or more software modules that implement the image quality enhancer IQE. The implementing software modules may be held in primary and/or secondary memory ME1.

Preferably, the data processing unit PU's implementing circuitry includes high performance processors PR1. Specifically, the processor(s) PR1 may be capable of parallel processing, such as those with multi-core designs to increase image-processing throughput. Specifically, in embodiments, graphical processing units GPU are used. Instead of, or in addition to, implementing the image quality enhancer IQE in software, hardware embodiments are also envisaged, such as FPGAs (Field Programmable Gate Arrays), ASICs (application-specific integrated circuit) or other soft- and/or hard-coded circuitry.

As will be explained more fully below, the image quality enhancer IQE is implemented by a machine learning ("ML") model G, previously trained on training data. The image quality enhancer IQE is thus operable in two modes, in a training mode and in deployment mode. In training mode, a training system TS, to be described in more detail below, is used to adjust parameters of an initialized model based on the training data to configure the trained model G. Once sufficiently trained, the so trained model G can then be made available for deployment, so that new imagery, not part of the training set, can then be processed during clinical use for example. Whilst the image quality enhancer IQE as described herein is mainly envisaged for processing imagery in image domain, processing of projection imagery in projection domain is not excluded herein, in particular if no-iterative reconstruction is to be used.

Whether in projection domain or in image domain, image values of an input image $I_{IN}$ to be processed may be thought of as encoding both, (true) structure and noise. This can be conceptualized additively as $I=S+N$, where $I$ indicates the actual image value recorded or reconstructed, S represents the structural signal and $N$ a noise contribution. The structural contribution S ideally represents aspects of the medical structure of interest in the object or patient imaged, whilst for $N$ there is no such correlation. It is an object of the image quality enhancer IQE to in particular decrease noise contribution, and hence increase the signal-to noise-ratio SNR. In addition or instead of improving signal to noise ratio, it is envisaged to improve contrast, in particular contrast versus noise ratio CNR. The image quality enhancer IQE may also act as a deblurer to reduce, or eliminate, image blur to so increase image sharpness. Increasing image quality is desirable as this can help clinicians to arrive at more accurate diagnostic or therapeutic conclusion.

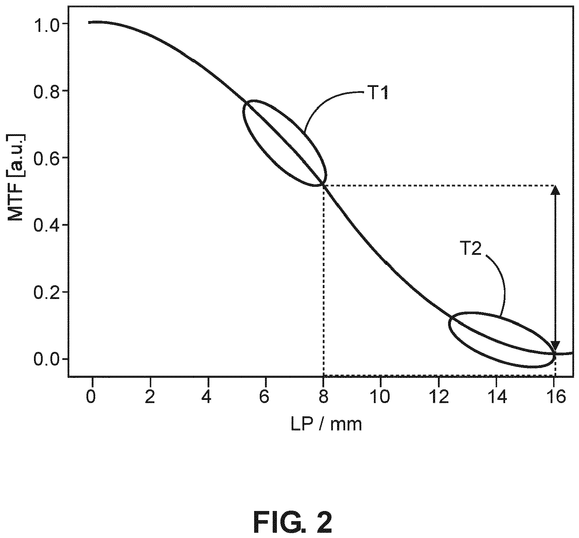

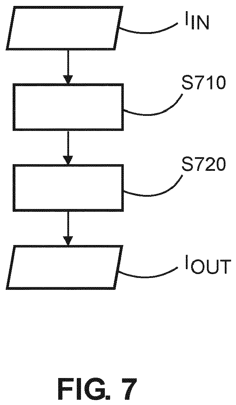

In the following, main reference will be made to the training aspect or training phase of the image quality enhancer IQE, whilst its deployment will be described with reference to Figure 7. Before providing more details on the training phase of the machine learning model G, reference is made first to diagram in Figure 2 to better motivate the approach proposed herein. Figure 2 shows an exemplary diagram of a modulation transfer function ("MTF") of a given image. The modulation ratio as expressed by the modulation function is shown on the vertical axis. It represents the discriminatory power of the imaging system versus structure shown in the horizontal axis as spatial frequency in lines per millimeter LP/mm. The modulation ratio as captured by the modulation function describes contrast fluctuations around a mean value and is a measure of how powerful the imaging system is in delivering contrast for ever finer structures, as one progresses along the spatial frequency axis. The modulation transfer function MTF may be understood as a Fourier-transformed point spread function (PSF) in spatial domain. Generally, the modulation ratio degrades with increased spatial frequency as represented by the MTF tail T2 of a certain input image. If one were to downscale the input image one will observe the phenomenon that the MTF tail T1 of the downscaled image is higher than the MTF tail T2 of the un-scaled, original input image. Having a higher MTF tail means better contrast delivery than in the un-scaled image, albeit over a reduced frequency range. In addition to the improved MTF behavior, the downscaled image also encodes less noise so has better SNR. Increasing the MTF, especially for higher spatial frequencies, allows boosting image sharpness.

It is proposed herein to use machine learning in order to propagate these improved IQ properties, that is reduced noise and improved MTF, as observed in downscaled imagery to the original non-scaled image. In other words, the machine learning model is trained herein so as to learn the relationship of the improved IQ in downscaled imagery versus the original imagery. The trained model G encoding this relationship can hence be applied to transform the original image to an IQ enhanced version thereof. This relationship can be thought of a latent mapping between the two image space, the downscaled image space and the original image space. It would appear difficult to learn this latent mapping analytical by using specific dependency assumptions. Advantageously, in ML, there is no need for such specific dependency assumptions. Indeed, in embodiments a machine learning network is used that is trained to "re-draw" a given input image in a "diction" of improved SNR and improved MTF as may be observed in downscaled imagery, to thereby arrive at a new version of the input image with higher IQ and yet natural appearance. It has been observed that existing image quality enhancers, whilst enhancing image quality, also introduce a rather artificial image appearance, which was not well taken up by clinical users who are used to a more "classical" look because of their education in medical school. With the proposed image quality enhancer, image quality can be improved without distorting unduly image appearance. The user will be awarded with a better image quality, but may still feel that he or she is looking at a "classical" image.

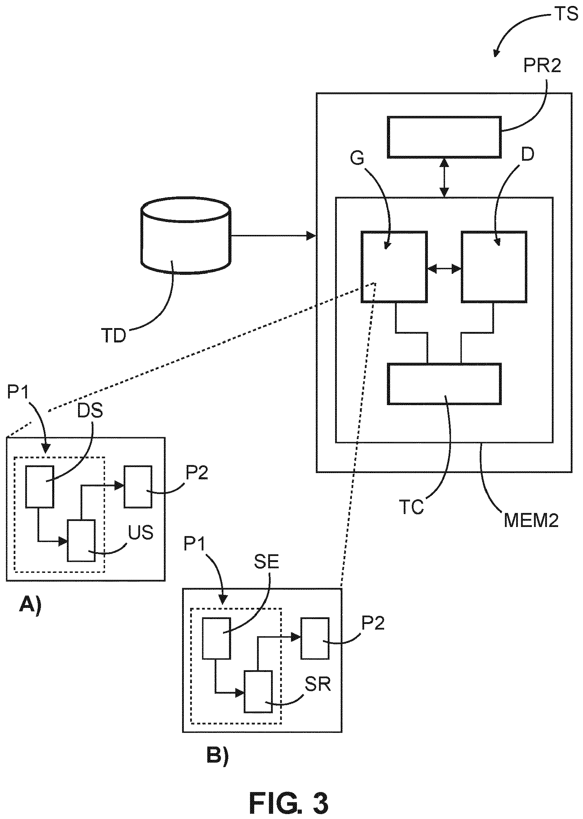

Reference is now made to Figure 3 which shows more details of the training system TS envisaged herein to train the machine learning model G. As can be seen, the target model G (the one we wish to train to enhance IQ) is embedded in a framework of additional one or more models which are trained together by operation of a training controller TC. The training system may be implemented in general on a computing device with processor PR2 and memory MEM2. The computing system configured to deliver the training system TS is in general different from the computing system that may be used during deployment. This is because computational demands for training are usually higher than during deployment and more powerful, and hence more expensive, computing equipment may be called for to perform the training. The training may be done repeatedly when new training data becomes available, or training can be done as a one-off operation, for example on setup. Once training phase has completed, the trained model G can be ported to a computing system PU with possibly lower computational power. The trained model G can then be applied by the image enhancer IQE during deployment to enhance new imagery that may emerge in clinical use.

As will be explained in more detail below, the training framework mainly envisaged used herein is that of a generative adversarial neural-network ("GAN")-type set up. This kind of neural-network setup includes a generator network G and a discriminator network D which are coupled together so that output of the generator G can be processed by the discriminator D. The generator G functions herein as the model of interest we wish to train for IQ enhancement. The generator network G and the discriminator network D are preferably arranged as artificial neural-networks of the feed-forward type or recurrent type. The generator G and the discriminator D architectures are stored in memory MEM2 as suitable data structures, such as matrices of two or higher dimension. The training data is held in a training data storage TD. Parameters of the training system, that is, parameters of the generator G and discriminator D, are adjusted, preferably iteratively, during training operation. The parameter adjustment is overseen and coordinated by the training controller TC. The training controller TC implements an objective function that processes data produced by the models G and D in response to training data. More specifically, the parameters of models G and D are adjusted so as to improve the objective function. The objective function may be formulated as a cost function, with the parameters to be adjusted so as to improve, that is to decrease, the values returnable by the cost function. Alternatively, the training scheme may be configured to increase a utility function. The training system iterates through a, preferably large, number of training data items which may include historic imagery. Preferably, the proposed system can be used in a non-supervised learning set-up so that the training data items may not need to be labeled beforehand, usually a tedious and expensive exercise. Training data may be obtained from historic imagery such as CT imagery of previous patients as held in PACS (picture archiving and communication system), or a hospital information systems (HIS) or other medical image repositories.

In-set Figures A, B show more details of the architecture of the generator network G envisaged herein. In order to configure for the above (at Figure 2) mentioned propagation property, suitable architectures envisaged herein may include two network portions P1 and P2. The propagation property discussed in Fig 2 relates to the network's ability to learn pertinent properties of downscaled images, that is, to extract therefrom the useful high tail MTF behavior and improved SNR and apply same to the input imagery.

In more detail, the first network portion P1 receives the training input image $\tilde{I}_{IN}$ and processes it into intermediate image S' which in turn is then processed by the second portion P2 to attempt estimating a higher image quality version $\tilde{I}_{OUT}$ of training input image $\tilde{I}_{IN}$ and provide this as training output. The tilde "~" notation will be used herein to indicate training input and training output data, as opposed to no-tilde notion for imagery processed during deployment in Figure 7. Also, for intermediate imagery that emerges "inside" the networks D,G during processing of training data, again no tilde designation will be used.

In general, neural-network type architectures include a plurality of computational nodes, which are arranged in cascaded layers. The artificial neural networks (referred to herein simply as "network(s)"), such as discriminator D and generator G are envisaged herein are deep networks in that they each include an input layer, an output layer and in between one, two or much more hidden layers. The training input imagery $\tilde{I}_{IN}$ is applied and processed at the input layer and then propagates as feature maps (more on which below) through the network from layer to layer, to then emerge at the output layer as training output imagery $\tilde{I}_{OUT}$, the estimate of enhanced training input image. Within the hidden layers, local input and output is usually referred to as the above mentioned feature maps. Feature maps produced by one layer are processed by the follow-up layer, which in turn produces higher generation feature maps, which are then processed by the next follow up layers and so on. The number of feature maps in each generation may grow from layer to layer. "Intermediate" images (also referred to herein as feature maps) as described herein include input/output produced within the neural network. In other words, there is at least one more layer which is to process the intermediate image.

The training input and output imagery as well as the feature maps may be represented and stored as matrices of two, three or higher dimensions depending on the number channels one wishes to process and the number of features maps to be produced. The features maps and the training input and output imagery can thus be said to have size, namely a width, height and depth, which represent the spatial dimension of the matrices. The output of the discriminator D may be represented in terms of a classification vector as will be explored in more detail below.

The processing by each hidden layer is a function defined by a set of numbers, also referred to as "weights" which are used to compute the output feature map in the given layer based on the received input feature map. These set of numbers are called filters. There may be more than one such filter per layer. A layer may thus produce more than one feature map. Specifically, the weights operate on a previous generation feature map to produce a logit z which is then passed through an activation layer of the given layer to so produce the next generation feature map. The operation on the previous generation feature map to compute the logit may be in terms of a linear combination of nodes of the previous layer and the said weights. Other functions may be used instead to compute the logits. The parameters may further include an additive bias term. The activation layer is preferably a non-linear function such as a soft- or hard-thresholder. Sigmoid functions, *tanh*-functions, *soft-max*-functions, rectified linear units "ReLU" (= max {$z$,0}, with $z$ the logit) may be used herein in the activation layer of an input, output or hidden layer.

In fully connected layers, each node is a function of all feature map entries of the previous layer. However, there are also a different type of layers, convolutional layers, for which this is not the case and the output feature map in the convolutional layer is produced from processing only a sub-set of entries/nodes of the input feature map received from the previous layer. The sub-sets so processed change in a sliding window manner for each logit in this layer, each logit being a function of a different sub-set of the input feature map. The sub-sets so processed preferably tile the entire previous feature map. This allows processing in a convolutional-style known from classical signal processing, hence the name "convolutional" layers. The step-width by which the processing window is slid over the current feature map is described by a hyper-parameter called the "stride". With stride equaling one, the size of the feature map as produced at the output of the convolutional layer is usually preserved, and equals that of the input feature map. Padding may be used to process entries close to the edges of the input feature map. Using stride equaling two or more allows reducing the size of the output feature map compared to the input feature map for a given convolutional layer. In this manner, a downscaling operation may be modelled as envisaged in embodiments to be described in more detail below.

De-convolutional layers, the operational quasi-inverse of convolutional layers, and are also envisaged in the training system TS in embodiments. De-convolutional layers allow up-scaling operations to be modeled. Interpolation techniques may be used to increase the size of the output feature map compared to the size of the input feature map.

Other functional layers may also be used herein such as max-pooling layers, drop-out layers, and others.

The sequence of cascaded hidden layers may be arranged in different network portions PI, P2 as explained above. Details of those network portions PI, P2 are now described in more detail. The downscaling operation as described above in Figure 2 can be thought of an operation to achieve a simplified representation of the imagery. The first network portion can hence be thought of as being configured to force the network to learn how training input imagery $\tilde{I}_{IN}$ transforms under simplification, and to then feed this knowledge into the second portion which is more concerned with learning noise behavior of the simplified imagery.

The simplifier portion P1 may include two processing strands or paths, each with their own cascaded layers. One path transforms input image $\tilde{I}_{IN}$ into the said simplified representation, and the other path then re-transforms the simplified representation back into a more complex version of the input image at a similar complexity as the input image. Thus, the two processing strands can be thought of as complexity reducers or "contactors" and complexity enhancers or "expanders", respectively, acting in sequence.

In one embodiment, the complexity reducer is implemented as a downscaling path DS, and the complexity enhancer is implemented as an up-scaling path US. In more detail, the down/scaler strand DS downscales the input image $\tilde{I}_{IN}$ to achieve an intermediate image of smaller size than the input image. The intermediate image is then processed by the up-scaler US in an attempt to recover an image version at a scale equaling that of the input training image.

In another embodiment, complexity of representation is not achieved through scale change, but through sparsity changes. Any image can be thought of as a representation of an instance in high dimensional space, for instance an image of size $n \times m$ ($n, m$ being the rows and columns layout of its pixels) is an element in an $n \times m$ dimensional vector space. In choosing such a representation one already imposes certain restrictions on what an algorithm can or cannot represent. Each element of a vector space can be represented as linear combinations of certain basis elements. An element is said to be sparser than another if its expansion in terms of basis elements has more zero coefficients. In other words, a representation of an element is sparser than another, if fewer such basis elements are used for the linear combination for that element. For instance, in the vector space of matrices, the basis elements are all possible matrices that can be formed with zero entries, safe for one entry being unity. An image of feature map is the sparser the more zero entries it has, or, more generally, the more entries it has below a certain negligibility threshold.

In embodiments as shown in inset B), instead of scaling up or down the input image, the two processing strands of the first maximum portion P1 are now arranged as a sparsity enhancer SE arranged in series with sparsity reducer SR which then feeds its output into the second portion P2. The sparsity enhancer SE processes the training input image to produce an intermediary representation at a higher sparsity than the input image. This intermediate higher sparsity image is then processed by the sparsity reducer SR to produce an output image to restore density to achieve a sparsity comparable to that of the input image. In particular over-complete representation with sparsity may be used. In this embodiments, the sparsity enhancer SE operates to increase the geometrical size of input image $\tilde{I}_{IN}$ and sparsity in the intermediate image. The sparsity reducer SR then reduces the size and sparsity to restore a more denser representation.

Other complexity reducing and restoring transformations/re-transformation networks are also envisaged herein in other embodiments for the first network portion P1.

The second network portion P2 that processes output by the first network portion P1 can be thought of as a noise learner that learns how noise features transform. Broadly, the noise learner PS2 compares the output of complexity learner PS1 with the original input image $\tilde{I}_{IN}$.

The objective function as implemented by the training controller TC is adapted accordingly to coordinate the processing of the two network portions PS1, PS2 as will be described below in more detail below.

It will be understood that, in terms of the above introduced terminology, the intermediate image in insets A), B) of Figure 3 is a feature map or may be combined from such feature maps.

In the embodiments of Figures 3 A) B), by propagating the input in a "detour" through the "code", overfitting can be reduced and learning improved.

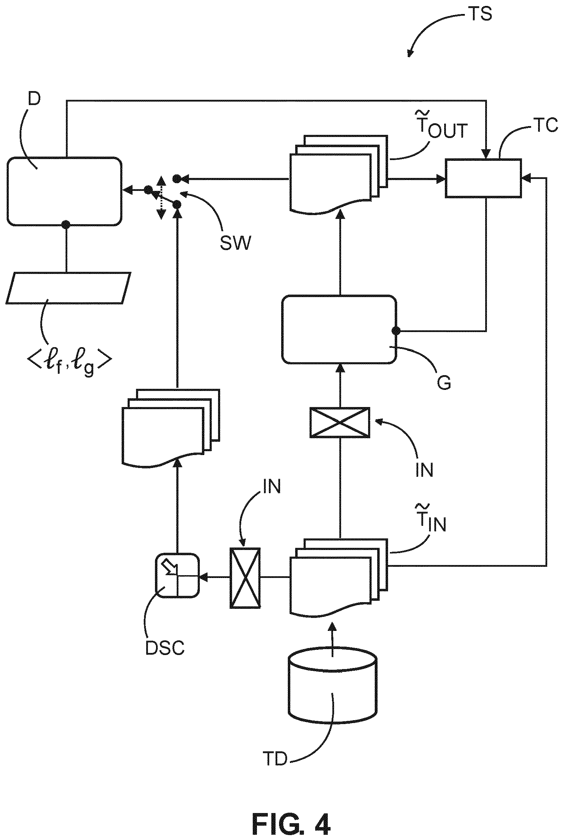

Turning now in more detail to the architecture of the training system TS in which the target network G is embed, reference is now made to Figure 4. As already mentioned, a generative-adversarial type architecture is envisaged herein. Previously, such architectures were reported by Ian Goodfellow et al in "Generative Adversarial Networks", published online 10 June 2014, available online under arXiv: 1406.2661.

In this or similar generative adversarial set-ups of networks (*"GANs"*), the generator network G, and the discriminator network D, are pitted against each other as controlled by cost function implemented by training control TC. For present purposes, the target network to be trained is the generator network G. In this adversarial set-up, the generator G processes imagery drawn from the training data TD and received at input port IN to produce the training output image $\tilde{I}_{OUT}$. The discriminator D processes this training output image and attempts to classify whether this image $\tilde{I}_{OUT}$ was in fact directly derived from the training data set or was artificially generated by the generator G.

Looked at from a probabilistic point of view, the generator G producing its output may be thought of as sampling from a first probability distribution *Pr1*. The training data in the training data set can be considered as samples from another probability distribution *Pr2*, the ground truth probability distribution. It is then an objective herein by the controller FC to adjust the parameters of the networks G,D so that the two probability distributions become indistinguishable for the discriminator D. The relationship between the generator G and the discriminator D can be understood in terms of a zero-sum game of game theory, because an advantage for the generator is at the detriment of the discriminator. In other words, the objective as expressed by the suitably configured cost function is to dupe the discriminator into believing that imagery produced by the generator during the training was drawn from the training data set. In other words, discriminator D can statistically not distinguish whether the image has been artificially produced by the generator G or was in fact drawn from the training data itself. The cost function used by the controller TC is configured to measure how the two probability distributions *Pr1, Pr2* differ from each other. The parameters are adjusted so as to decrease this measure, thus increasing the statistical indistinguishability. Suitable measures that may be incorporated into the cost function as terms include a cross-entropy term or, more generally, a Kullback-Leibler divergence (KLD) measure. Other statistical measure terms may be used that allow measuring a distance between probability distributions. A suitable cost function will be discussed in more detail below.

In the training system TS, a deterministic or random switch SW is used that allows selectively feeding different input images into the discriminator, either drawn direct from the training set TD, or by switching to pick up the generator G's output $\tilde{I}_{OUT}$. Accordingly, the discriminator D attempts to classify the output $\tilde{I}_{OUT}$ into one of two classes, genuine imagery $\ell_g$ drawn from training set TD, or "fake" imagery $\ell_f$ as produced by the generator G in its operation.

In order to configure the training system TS to propagate the useful MTF and noise behaviors as explained above at Figure 2, a down-scaler DS is interposed between the discriminator D and the training data set TD. In other words, it is not the original sample drawn from the training data set that is provided to the discriminator by switch SW, but the drawn training sample is first downscaled and it is this downscaled version that is then provided through switch SW for the discriminator D to classify. The down-scaler DSC is useful because it allows the removal of the high frequencies of the image. The removed high frequencies usually have low MTF values, and the left high frequencies in the image usually have higher MTF values.

The down-scaler DSC may be implemented by any known downscaling algorithm, such as skipping of pixels or voxels or any other, thereby reducing the size of the image. Interpolation methods may be used, such as bilinear interpolation, bi-cubic interpolation or spline interpolation, or others still. The downscale operation by down-scaler DSC may change during the iterations. For example, in one instance, the down-scaler DSC may sample each *n*-th pixel from the training data image drawn through input port IN, whilst at the next instance, when drawing the next training image, a different sampling pattern is used, such as each *m*-th pixel being sampled, with $m \neq n$. Thus, the size of the down-scaled image version for the classification may vary. Changing the downscale operation may add perturbations during training, and this may help the training algorithm to converge to a more robust solution.

The training output image $\tilde{I}_{OUT}$ and the discrimination result produced by the discriminator D are then fed into the cost function as administered by the training controller TC to return a cost. Based on the cost TC, the parameters of one or both of the two networks D,G are then adjusted to reduce the cost. This is repeated in an iterative fashion until sufficient convergence has been achieved, that is, until for a sufficient number of training images the cost has dropped under a threshold.

Iteration may proceed in two loops, an inner loop and an outer loop. In the inner loop for any given data training image $\tilde{I}_{IN}$ that is processed, the model parameters are adjusted in one or more iteration cycles until a stopping condition is fulfilled. The switch by discriminator feed switch SW occurs preferably in iteration cycles of the outer loop, whilst no switching occurs in the inner loop during parameter update. In the inner loop, the discriminator D and generator G may be trained in alternating manner, i.e., one or more iterations to train the generator G, followed by one or more iterations to train the discriminator D. Processing by training systems may then switch into the second, the outer loop, in which a new training data set is then drawn, and processed as described above. The inner iteration loop is then re-entered into as described above, but this time it is the accumulated cost for the current and some of all previously processed training images that are considered when adjusting the parameters. The parameters as evaluated by the cost function may be adjusted, based on the backpropagation algorithm or any other gradient or non-gradient based numerical optimization method. The evaluation of the cost function may be implicit by configuring a suitable parameter updating/adjustment routine as is done in the backpropagation algorithm.

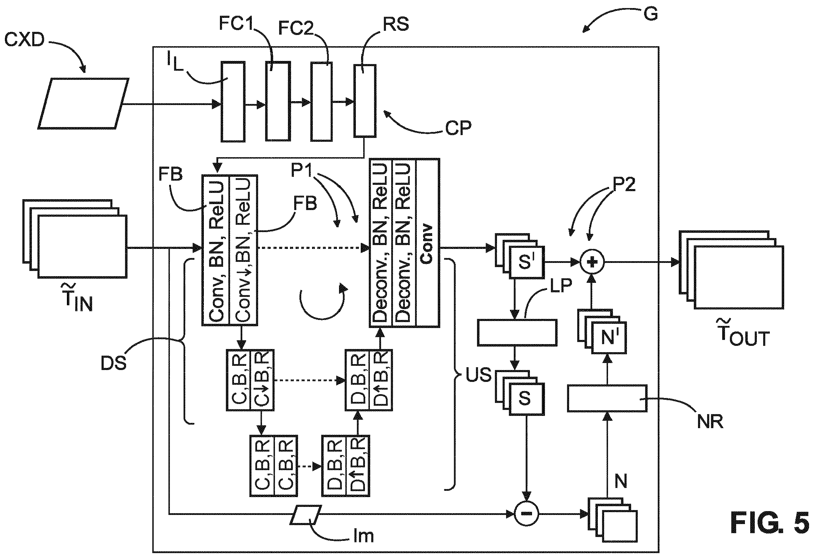

The generator G may not only be configured to process image data as previously discussed but may in addition be able to process contextual non-image data CXD, such as patient data and other to improve learning. Accounting for contextual information may lead to more stable, robust learning results. The processing of contextual data CXD will be described in more detail below at Figure 5.

It will be understood that once sufficiently trained, it is only the generator network G that is of interest herein, whilst the others network parts of training system TS, in particular the discriminator D, are of lesser interest herein and not required for image enhancement. In other words, once training has concluded, the current set of parameters of the network G in its current architecture can be copied and made available for image quality enhancement in clinical deployment. The training may be continued later with new training data.

The generator network G, in particular the simplifier portion P1 and the noise learner P2, may be arranged as fully convolutional layers, without fully connected layers. Hybrid version are also envisaged herein where fully connected and convolutional layers are jointly used, and so is the use of exclusively fully connected layers. However, for the processing of image data, preferably fully convolutional layers are used, whilst for the processing of the context data CXD fully connected layers are preferred.

Broadly, the generator is a regressional type network, regressing its input image $\tilde{I}_{IN}$ into its output image $\tilde{I}_{OUT}$. In contrast, the discriminator network D is configured as a classifier and may be arranged as a network with one, two or more fully connected layers that process the discriminator input image to produce at its output layer the classification result $<\ell_f \ell_g>$. Optionally, one or more convolutional layers may be used. The discriminator input image is either the generator output image $\tilde{I}_{OUT}$ or the downscaled input image $\tilde{I}_{IN}$ as provided by switch SW. The classification result may be provided at discriminator D's output layer as a vector with two entries representing respective probabilities for the two labels

$< \ell_f, \ell_g>$ as explained above. The discriminator D output layer can thus be configured as a combiner layer that combines the previous feature maps into normalized output of two numbers between, and including, zero and one. In embodiments, a soft-max layer may be used that allows inter-layer processing in the output layer to produce the normalized result. This is different from the hidden layers, which generally process exclusively feature maps from the previous layer and not output from nodes within the given layer.

In preferred embodiments, the discriminator G has a Markovian character. The classification operates per patches, or subsets that tile the discriminator input imagery, rather than classify the input image as a whole. Such a discriminator models the image to be classified, either $\tilde{I}_{OUT}$ or its the downscaled version of $\tilde{I}_{IN}$, as a Markov random field, assuming independence between pixels separated by more than a patch diameter. See P Isola et al in "Image-to-image translation with conditional adversarial networks", published in "Proceedings of the IEEE conference on computer vision and pattern recognition", pp. 1125-1134 (2017). The advantage of using such a Markovian discriminator is modelling of spatial high-frequencies. Specifically. the Markovian discriminator D classifies each of *N x N* patches as real or fake, so operates patch-wise rather than globally on the whole image at once. The patches can be made much smaller than the discriminator input image. The classification is repeated for different patches until the whole image plane is covered. A respective local classification result $<\ell_f, \ell_g>^i$ is computed per patch *i*. The local classification results may be consolidated for example by averaging to obtain a global classification result/label $<\ell_f, \ell_g>$ for the whole image. This global classification result is then fed to the training controller TC for cost function evaluation and parameter adjustment.

It will be understood that in the proposed GAN-based training system TS, no labelling of the training data is required. By adjusting the parameters so that the discriminator fails to distinguish the two classes $<\ell_f, \ell_g>$, the training of the target model, the generator G, happens automatically.

An embodiment of regressional generative layer G for the down and up-scaler embodiment in part A) of Figure 3 is now discussed in more detail in block diagram Figure 5, to which reference is now made. The contractor and expander portions of network section P1 of generator G may be arranged in embodiments as a multi-scale network, similar to the *U-net*-type architecture described by Ronneberg et al in "U-Net: Convolutional Networks for Biomedical Image Segmentation ", published in N Navab et al (eds), "Medical Image Computing and Computer-Assisted Intervention -MICCAI 2015", "Lecture Notes in Computer Science", vol. 9351 (2015), Springer, Cham.

In the diagram of Figure 5, functional layers such as convolutional ("*C*", "*Conv*"), de-vonvolutional ("*D*", "*Deconv*"), batch normalizer ("*B*", "*BN*") and activation (*"RELU"*), have been arranged in functional blocks FB, although this is merely for ease of representation. In the embodiments, the activation layers are arranged as a RELUs but this is exemplary and other activation layer types, such as sigmoid or *tanh*-function may be used instead as described above. The arrow symbols pointing up and down "↑", "↓" in Figure 5 are indicative of up-sampling or down-sampling operation, respectively of the de-convolutional layer or the convolutional layer. The up-sampling operator can be configured as a de-convolutional layer with a suitable stride. The down-sampling operator can be arranged as a convolutional layer with stride greater than two. Whilst pooling layer could also be used for down-sampling, convolutional layers with stride greater two are preferred instead. The greater the stride, the greater the downscaling. One or more drop-out layers may be used that randomly sever connections between layers to reduce the risk of over-fitting. The batch normalizer perform adjustments of weights in a layer to avoid vanishing gradient problems as may happen in backpropagation and similar types of gradient based approaches that rely on the chain rule for formulation.

The contractor strand DS is arranged as the down-sampler path DS as per A) in Figure 3 above, which processes input training image $\tilde{I}_{IN}$ to produce, over a number of scales, the intermediate image $I_{im}$ at a lower scale than that of the input image $\tilde{I}_{IN}$. This intermediate image $I_{im}$ is also sometimes referred to herein as "the code" as it is thought to "encode" features of image $\tilde{I}_{IN}$ at a lower scale. A three-scale network with three scaling levels are shown in Figure 5 represented by the three dashed arrows, but more or less scaling levels may be used instead. The contractor strand DS may be implemented using a repeated application of two (or more) convolutions, optionally each followed by a batch normalization BN, and a rectified linear unit (ReLU) or other activation function. The convolutional layers may be implemented by 3x3x3 filters. Larger or smaller filters my be used instead. The last convolution of each scale level in each functional block FB in contractor strand DS may be implemented with stride 2 for down-sampling/downscaling.

In the expander path or up-scale strand US, the code $I_{im}$ is sequentially scaled back up to the same scale layer as the input image $\tilde{I}_{IN}$, and is released as output S' for processing by noise learner network section P2. Optionally, feature maps from the down-scaling path DS may be fed as additional input at the corresponding scaling level into the up-scale path, as shown schematically by the three dashed arrows running from left to right. These cross-inputs may be processed as additional feature maps in additional channels in the respective layer at the corresponding upscale level in the expander path US. This cross-feeding across scales, also called skip-connections, allows for better localization. In general, up-sampling learning can be improved by providing additional information, such as through the said skip-connections. The cross-fed information may help the up-scale path US to better recover, that is, localize, even smaller image features.

The expander strand US may be implemented by repeated application of two (or more) deconvolutions, each followed optionally by a batch normalization and a ReLU or other activation function. The last deconvolution of each scale is done with stride 2 for up-sampling/upscaling. The deconvolutions may be implemented as 3x3x3 filters, but smaller or larger filters are also envisaged.

In general, the number of scales in the two strands US, DS are equal. At the final layer in the expander path US, a 1x1x1 convolution may be used to provide the final output S' image of network portion P1.

As mentioned, given convolutional/deconvolutional layer may use more than filter. A typical number of convolution/deconvolutional filters in each layer in scale level s is $(2^3)^s C$, and wherein $s$ = 1,2,3, ... is the scale level. The initial input is $\tilde{I}_{IN}$ is considered to have scale equal 1. $c$ is a network control parameter. For setups with over complete representation, $c > 1$ is preferred.

Whilst the size of the feature maps decrease during propagation through the down scale path DS all the way down to the code $I_{im}$, this is reversed and the size increases as the code $I_{im}$ progresses through the upscale path US to produce the next intermediate image S'. S' has preferably the same size and has the same scale as the input image $\tilde{I}_{IN}$. It is preferred herein that the number of feature maps, and hence the number of convolutional filters used in the layers, increases in the down scale DS path, whilst the said filter number may decrease in the up-scale path US.

The up-scaled intermediate image S' is passed on to the noise learner section P2 to be processed there, as will be now explained in more detail with reference to the right portion of Figure 5. In embodiments, a low pass filter LP, such as normalized convolutional layer, is applied to the intermediate image S' to obtain a sharpened version S, an intermediate structure image, which can be thought to encode more structure than noise. Specifically, the noise level is expected to be absent or negligible in intermediate structure image S'. The normalized convolution layer may be configured as a single channel layer without bias in which the sum of the kernel weights equal unity. The structure image S so obtained, which has the same size as the input image $\tilde{I}_{IN}$, is then subtracted point-wise from the input image $\tilde{I}_{IN}$ to produce an intermediate noise image N, that is, an image including more noise than structure. This intermediate noise image N is an estimate for the noise level in the input image $\tilde{I}_{IN}$. This intermediate noise image N is then noise-reduced by noise reducer NR, for instance by scalar multiplication with a number less than unity, to produce an intermediate image with reduced noise N'. The intermediate image with reduced noise N' is then added to the intermediate output S' of previous network section P1, to so produce the estimated training output image $\tilde{I}_{OUT}$. The noise N' is thus the final noise estimate that is included into the final output image $\tilde{I}_{OUT}$. Because of the noise reducer NR, the final output image $\tilde{I}_{OUT}$ is noise reduced relative to the input image $\tilde{I}_{IN}$, but the now (re-)included reduced noise estimate ensures a more natural, classical appearance similar to a scaled-down version of the input image $\tilde{I}_{IN}$. In practice, Applicant has observed that the removal of too much noise cause a synthetic and unnatural look, as mentioned above.

In sum, the transformer/re-transformer network portion P1 learns a sharpened intermediate image S' with no, or only negligible, noise. The intermediate image S' is sharpened relative to the input image $\tilde{I}_{IN}$ The noise learner P2 learns, based on the intermediate image S' and the original input image $\tilde{I}_{IN}$, a reduced noise estimate N', which is then added back to the intermediate image S' to obtained a noise reduced and yet natural looking version $\tilde{I}_{OUT}$ of the input image $\tilde{I}_{IN}$. By learning noise features in the noise learner portion P2 and by learning the scaling behavior in the first portion P1, the propagation property as discussed above in Figure 2 is achieved by forcing the network G to learn the manner in which information transforms under downscaling, thus securing the advantageous MTF and noise behaviors.

As shown in the upper left portion of generator network G in Figure 5, in addition to the two network portions PI, P2, the generator may G may further include a network portion CP capable of processing non-image contextual data CXD. As mentioned briefly above, the contextual data CXD may include any one or more of i) patient bio-characteristics, ii) specifications of the image acquisition process and iii) specifications of the image reconstruction process. The patient bio-characteristics may include patient medical history, patient age, sex, weight, ethnicity etc. The specifications of the image acquisition process may include acquisition imaging parameters, such as any one or more of scan type, body part scanned, X-ray tube voltage kVp and amperage mA, radiation produced mAs, rotation time, collimation and pitch. The specifications of the image reconstruction process may include any one of more of reconstruction filter, reconstruction algorithm (e.g., FBP, iDose or IMR), slice thickness, slice increment, matrix size and field of view.

In order to process this type of mainly non-image data CXD, the context data processor network portion CP may be arranged as a separate strand of cascaded fully connected hidden layers FC1, FC2. Only two are shown, but there may be merely one or more than two. One-hot encoding may be used to encode context data CXD as vectors or matrices. In addition or instead, an auto-encoder network may be used where the data CXD is transformed into a code at the center portion of the auto-encoder to obtain a simplified code in a denser representation, as the one-hot encoding of the contextual data is likely to be sparse which may be undesirable for good processing. A re-shaper layer RS as the final layer of the context processor network CP ensures that the contextual data CXD is processed into output, represented as one or more matrices that correspond in size to the input imagery of network portion P1. In this manner, the contextual data CXD is effectively transformed by network portion CP into "pseudo images" which can be fed into the network, for instance, in a separate image channel(s), and can be so processed alongside with the input imagery. The contextual data in form of pseudo images is hence mixed as a new channel into the image data for joint processing. The pseudo imagery for the contextual data CXD is not necessarily fed into the input layer of network portion P1, but may instead be fed into other positions of the network, suitably transformed by reshaper RS in shape and size to be mixable as an additional channel input at the respective scale level into the network portion P1.

The reshaper RS may reshape the output of the last fully connected layer to a pseudo volume or number of such pseudo-volume representation. In embodiments, the size of each volume is the size of the input volume (input images) $\tilde{I}_{IN}$ or that of the feature map at the scale level $s$ where feed-in is intended. Reshaping may be done by populating each output value of the last fully connected layer to a separate volume of intended size, where the entire pseudo-image volume is filled-up by the respective output value.

The U-net type architecture as shown in Figure 5 is according to one embodiment, and others are also envisaged. The skip connections across scales are optional. If skip connections are used, it is not necessary to use same for all scale levels. Instead of the U-net setup, an auto-encoder network architecture could be used for the processing of image data in generator G.

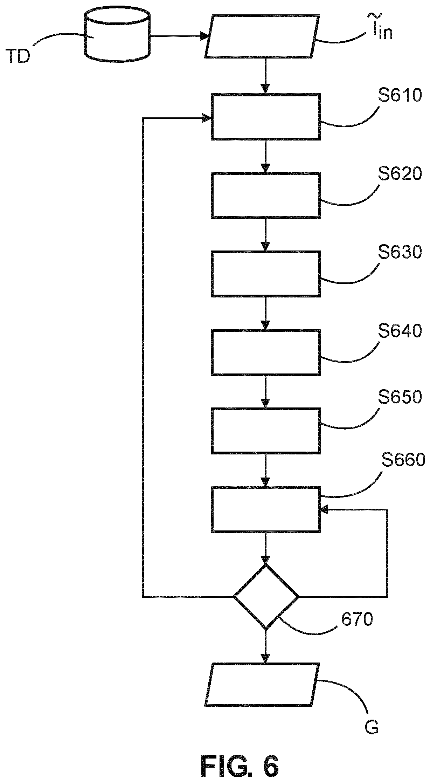

Reference is now made to Figure 6, which shows a flow chart of a method of training an ML network for image quality enhancement. The method may be used to implement the above described training scheme TS. However, it will be understood that the steps of the method are not necessarily tied to the architecture discussed above.

At step S610, a training input image $\tilde{I}_{IN}$ is received from a training data set. The training data set may be obtained from historic databases with relevant imagery.

For training, a framework of an artificial neural network of the generative-adversarial (GAN)-type is used. The network architecture includes a generator network and a discriminator network.

At step S620 the generator network processes the training input image $\tilde{I}_{IN}$ to produce a training output image $\tilde{I}_{OUT}$.

At step S630 the input image is downscaled.

Per iteration cycle of the outer loop in which training data set is accessed, it is either the said downscaled image or the training output image that is provided through a switch to the discriminator as input for classification. The switch may be random or may be deterministic, so as to follow a predefined switch pattern, such as alternating between training data set and generator output.

At step S640 the discriminator attempts to discriminate between the two images by classifying the input image accordingly as i) an instance $\ell_g$ of a member drawn from the training data set or ii) as an instance $\ell_f$ of output by the generator. The classification constitutes an attempted discrimination result that may or may not be correct.

At step S650 the discrimination result and the training output image are fed into a cost function that controls the training procedure. The cost function evaluates the discrimination result and the output image. Based on the evaluation, the current parameters of the generator and/or discriminator are updated or adjusted at step S660 to improve the cost function. The cost function takes into account all or some of the previously processed inputs from previous outer loop cycles so that it is the accumulated cost that is improved when adjusting or updating the parameters. The parameter adjustment may be done in one or more iteration cycles. During the inner loop iteration, the switch is preferably not operated. At step S670 a stopping condition for the inner loop iterations is evaluated. The stopping condition may be set as one of a fixed number of inner loop iterations or as a condition of convergence within a defined deviation margin. Once it is determined that the stopping condition is fulfilled, method flow exits the inner loop, and the outer loop is (re-)entered into. Flow returns to step S610 where a new training input image is accessed and processed as described.

If it is determined that the stopping condition not fulfilled, iterations in the inner loop continue to improve the cost function by parameter adjustment.

Once all or a pre-set number of training data items have been processed, the generator network G with the current parameter set considered sufficiently trained and can be made available for deployment. Alongside and in addition to processing the image data the contextual data may be processed as described above.

The training controller TC administers cost function evaluation, explicitly or implicitly, by implementing a parameter update function. Specifically, the evaluation of the cost function may be implicit in the manner in which the parameters are updated. The structure of the cost function and the specific optimization algorithm applied to the cost function often yields an update function that is guaranteed to improve the cost function and the evaluation is implicit in the manner in which the update is done. Such is the case for example in backpropagation methods with its sequence of repeated forward and backward passes. In other, although less efficient, brute force optimizations, the cost function is explicitly evaluated after each parameter adjustment. The adjustments are in suitably small steps in parameter space either in random directions, or along the gradient of the cost functions such as in Newton-Raphson-type numerical methods, if the gradient can be computed in good approximation and gradient evaluation is tractable.

In the following, a suitable cost function $E$ formulation is described that is configured to enforce learning of the propagation property as discussed above at Figure 2:

$$\min_{\theta_G} \max_{\theta_D} E(\theta, I^*, \tilde{I}_{IN}) = \sum_{i=1}^{n} (\text{T1}(D(I^*), G(\tilde{I}_{IN})) + \lambda_N T2 + \lambda_{S'} T3 + \lambda_c T4)$$

$$(1)$$

The summation $i$ is over training input images $\tilde{I}_{IN}$ from training data set TD. $\theta$ are the parameters for the generator G and discriminator D to be learned, wherein $\theta_D$ are the parameters of discriminator D and $\theta_G$ are the parameters of generator G, $\theta = \{\theta_D, \theta_G\}$. $\tilde{I}_{IN}$ is the training put image , and discriminator input image $I^*$ as provided by the switch is either $I'$, a notation for the downscaled version of $\tilde{I}_{IN}$ as produced by operation of downscaler DSC, or $I^* = \tilde{I}_{OUT}$, the output of $G(\tilde{I}_{IN})$. $D(I^*)$ is either one of labels $<\ell_f, \ell_g>$, the discrimination/classification result as provided by discriminator D, based on the input received from switch SW.

The mini-max optimization of system eq(1) may be run as two optimizations alternating. This alternate optimizing corresponds to the adverse relationship between generator G and discriminator D. Specifically, eq(1) may be minimized with respect to the generator parameters $\theta_G$ whilst keeping the parameters $\theta_D$ of the discriminator D at their current values. Dual thereto, eq (1) may be maximized with respect to the discriminator parameters $\theta_D$, whilst keeping the parameters $\theta_G$ of the generator G at their current values. The parameters may be adjusted on an optimization algorithm administered by controller TC. This algorithm may be iterative. In the present disclosure, the term "optimization" does not necessarily mean that there is convergence to a global optimum (a minimum or maximum). A local optimum may be sufficient for some purposes. Iterations may be aborted based on a stopping condition before attainment of the local or global optimum.

Term *T1* is a function of the generator *G* output and the discriminator D's classification result. The term T1 may be configured to model the difference between the two probability distributions, the ground truth probability distribution *Pr1* and probability distribution *Pr2* for the generator G generated "fake" samples. The term T1 may be configured as a cross-entropy term. Any other measure may be used for this probability distribution comparison term such as KLD or other. Specifically, the term T1 is configured to train the discriminator to increase the probability of assigning the correct label to the input images it classifies. At the same time, and opposed to this objective, generator is trained so as to increase the probability that the discriminator classifies wrongly.

The terms T2-T4 are regularizes that are configured to enforce certain properties of solutions to minimization problem (1). Convergence behavior of the optimization algorithm can be controlled by the regularizer terms T2-T4. Optimization algorithm convergences to solutions having the respective property. Specifically, the regularizer terms are now described in more detail:

The term T2 is configured to enforce noise estimates *N* that are small compared to noise on the input image $\tilde{I}_{IN}$. *N* is enforced to represent noise in the input image.

The term T3 is configured to enforce structure estimate S' has greater sharpness than input image $\tilde{I}_{IN}$. Preferably, T3 acts as a regularizer to enforce that *S'* includes no, or only negligible, noise. T3 may be implemented as a term that favors smoothness. The term T4 is configured there is low dependency between structure estimate S and noise estimate N as computed by noise learner network portion P2.

Not all terms T2-T4 are necessarily required, and any combination of term T1 with any sub-selection of one or more of terms {*T2,T3,T4*} are also envisaged herein.

The $\lambda$'s are weights that model the relative strength or preponderance among the constituent cost function terms $Tj$, $_{j=1-4}$.

In more detail and in embodiments, the following cost function $E$ is used, with terms corresponding to T1, T2, T3 and T4 in this order:

$$\min_{\theta_G} E(\theta, I) = \sum_{i=1}^{n} \left( \log\left( 1 - D\big( G(I_i) \big) \right) + \lambda_N \left( \frac{N}{\hat{N}} \right)^2 + \lambda_{S'} R(S') + \right.$$

$$\left. + \lambda_c |C(S, N)| \right) \quad (2a)$$

$$\max_{\theta_D} E(\theta, I^*, I) = \sum_{i=1}^{n} \left( \log\big( D(I_i^*) \big) + \log\left( 1 - D\big( G(I_i) \big) \right) \right) \quad (2b)$$

wherein:-

$\theta_{G,D}$ are the networks parameter in particular for discriminator and generator networks D,G, respectively, $\theta = \{\theta_D, \theta_G\}$;

$I = \tilde{I}_{IN}$ is a given input images/volumes from the training set,

$I'$ is the set of downscaled images/volumes of $I$, obtained by operation of downscaler DSC

$G$ is the generator network, with $G(I)$ being the respective training output image $\tilde{I}_{OUT}$ for a given training input image $I = \tilde{I}_{IN}$;

$I^*$ is either $I'$ or $G(I)$, as provided by the switch;

$D$ is the discriminator network, with $D(I^*)$ being the classification result $<\ell_f \ell_g>$ .

$\lambda_N$, $\lambda_{S'}$ and $\lambda_c$ are control parameters;

$N, S'$ and $S$ are the intermediate results of the network $G$ as per Figure 5;

$\hat{N}$ is a noise map of $I = \tilde{I}_{IN}$;

$R(\cdot)$ is a roughness penalty or regularization term, for example total variation, Huber loss or other function; and

$C(\cdot)$ is the correlation or covariance function or any other measure for dependency.

For clarity, in eqs (2a.b) the min-max formulation of (1) has been split up in two optimizations eq (2a) and eq (2b) that alternate as described above. In eqs (2a,b), as an example, the term T1 is a binary cross-entropy based measure, $logD(\cdot) + log(1 - D(G(\cdot)))$, but other measures may be used instead.

Optionally and preferably, in eqs (1,2) above, the sub-images, or "patches", $p_j \subset I^*$ are provided as input for the discriminator (rather than the input image $I^*$ as a whole) for patchwise discrimination according to the Markovian embodiment as described above.

The noise map $\hat{N}$ to estimate noise contribution in the input image $\tilde{I}_{IN}$ per pixel may be computed as previously described by Applicant's US 8,938,110. The noise contribution $\tilde{I}_{IN}$ for each pixel can be estimated by computing the standard deviation ("std") in small neighborhood for each pixel position. Preferably, a smoothing filter such as wide-median filter may be applied after the local std filter to remove poor noise estimates at edge portions. Pixel values in the noise map $\hat{N}$ quantity the noise contribution at the respective pixel, whilst the noise image N includes contributions from noise and structure.

It will be understood that the terms in (2) are specific embodiments of *T1-T4* and other, alternative configurations are also envisaged herein.

In addition, the training could be boosted by using the Auto-mixing method as has been described by M Freiman et al in "Unsupervised abnormality detection through mixed structure regularization (MSR) in deep sparse Auto-encoders", published in "Medical Physics", vol 46(5), pp 2223-2231 (2019) or in Applicant's WO2019/229119.

Various GAN specific optimization algorithms are also envisaged, such as described by I Goodfellow et al in "Generative adversarial nets", published in "NIPS'14:Proceedings of the 27th International Conference on Neural Information Processing Systems", vol. 2, pp. 2672-2680, (2014), or by MArjovsky et al in "Wasserstein GAN", published as arXiv preprint on arXiv: 1701.07875 (2017), or by Guo-Jun Qi in "Loss-sensitive generative adversarial networks on Lipschitz densities", published as arXiv preprint on arXiv: 1701.06264 (2017).

If the embodiment of Figure 3B) is used, with sparsity enforcement, eqs (1), (2a) may include sparsity enforcement terms, such as those that penalize large values. See for example *Freiman et al* cited above, for example eq (5) page 8, or similar.

Reference is now made to Figure 7 which shows a slow chart of a method of computerized image quality enhancement. The method is operable once the generator model G has been sufficiently trained as described above.

At step 710 an input image $I_{IN}$ is received. For example, the input image $I_{IN}$ may be obtained during clinical use of an imaging apparatus IA.

This input image $I_{IN}$ is processed by the trained generator model G at step S720. Specifically, the input image $I_{IN}$ is applied to the input layer of the network and is propagated there-through to provide at the output layer of model G a quality enhanced version $I_{OUT}$ of the input image $I_{IN}$. The output image $I_{OUT}$ can be displayed on a display device DD, stored in a memory, or may otherwise be processed.

The user may furnish by a suitable input user interface contextual data CXD in relation to the image $I_{IN}$ to be enhanced. The contextual data CXD is processed at step S720 by network G jointly with the input image.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to device type claims. However, a person skilled in the art will gather from the above description that, unless otherwise notified, in addition to any combination of features belonging to one type of subject matter, also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or multiple processor, other computational unit may fulfill the functions of several items re-cited in the claims. The mere fact that certain measures are re-cited in mutually different dependent claims does not indicate that a combination of these measures cannot be used to advantage. Any reference signs in the claims, either numeric or alphanumeric, or a combination of one or more capital letters, should not be construed as limiting the scope.

**Claims**

1. A training system (TS) for training a machine learning model for image quality enhancement in medical imagery, comprising:

    an input interface (IN) for receiving a training input image ($\tilde{I}_{IN}$);
    an artificial neural network model (G,D) framework of the generative adversarial type including a generator network (G) and a discriminator (D) network;
    the generative network (G) to process the training input image to produce a training output image ($\tilde{I}_{OUT}$);
    a down-scaler (DS) configured to downscale the training input image, and the discriminator attempting to discriminate between the downscaled training input image ($I'$) and training output image to produce a discrimination result ($\ell_f, \ell_g$), and
    a training controller (TC) to adjust parameters of the artificial neural network model framework based on the discrimination result ($\ell_f, \ell_g$).

2. The system of claim 1, wherein the discriminator (D) wherein the discriminator (D) is configured to discriminate patch-wise.

3. The system of any one of the previous claims, wherein the generator (G) includes a first portion (P1) having a multi-scale architecture with two processing strands, a down-scale strand (DS) and an upscale strand (US), the down-scale strand to downs-scale the input image to obtain a first intermediary image, and the upscale strand to upscale the intermediate image to obtain the training output image ($I_{OUT}$) or a second intermediate image ($S'$) processable into the training output image.

4. The system of any one of the previous claims, wherein the generator (G) includes a first portion (P1) having an architecture with two processing strands, a sparsity enhancer strand (SE) and a sparsity reducer strand (SR), the sparsity enhancer to process the input image to obtain a first intermediary image with greater sparsity than the input image, and the sparsity reducer to reduce sparsity of the intermediate image to obtain the training output image or a second intermediate image ($S'$) processable into the training output image ($I_{OUT}$).

5. The system of any one of claims 3 or 4, wherein the generator (G) includes a second portion (P2) configured to process the second intermediate image ($S'$) into a third intermediate image ($N$), to reduce noise in the third intermediate image, and to combine the noise reduced noise image (N') so obtained with the second intermediate image ($S'$) to obtain the training output image.

6. The system of claim 5, wherein operation of the training controller (TC) is to adjust the parameters based on any one or more of i) the third intermediate image ($N$) versus a noise map ($\hat{N}$) computed from the input image ii) a smoothness of the second intermediate image property ($S'$), iii) a dependency between a) a low-pass filtered version ($S$) of the second intermediate image ($S'$) and b) the third intermediate image ($N$).

7. A trained machine learning model (M) obtained as the generative network (G) of the training system (TS) as per any one of claim 1-6, after processing one or more training input images.

8. A trainable machine learning model (G) including:

    a first portion (P1) having a multi-scale architecture with two processing strands, a down-scale strand (DS) and an upscale strand (US), the down-scale strand to downs-scale an input image to obtain a first intermediary image, and the upscale strand to upscale the intermediate image to obtain a second intermediate image ($S'$); and
    a second portion (P2) configured to process the second intermediate image ($S'$) into a third intermediate image ($N$), to reduce noise in the third intermediate image, and to combine the noise reduced noise image (N') so obtained with the second intermediate image (S') to obtain the training output image.

9. A method of training a machine learning model for image quality enhancement in medical imagery, the machine learning model being a generator network (G) of an artificial neural network model (G,D) framework of the generative adversarial type, the framework further including a discriminator (D) network, the method comprising:

    receiving (S610) a training input image ($\tilde{I}_{IN}$);
    processing (S620), by the generative network (G), the training input image to produce a training output image

$(\tilde{I}_{OUT})$;

downscaling (S630) the training input image;

using the discriminator network (D) to attempt discriminating (S640) between the downscaled training input image ($I'$) and training output image to produce a discrimination result ($\ell_f, \ell_g$), and

adjusting (S660) parameters of the artificial neural network model framework based on the discrimination result ($\ell_f, \ell_g$).

10. The method of image quality enhancement in medical imaging, comprising:

receiving (S710) an input image; and

applying (S720) a trained machine learning model (G) as per claim 7 or 8 to the input image to obtain an output image.

11. An imaging arrangement (IAR), comprising an imaging apparatus (IA) and a computing system (PU) that implements a model as per claims 7 or 8.

12. The imaging arrangement of claim 11, wherein the imaging apparatus (IA) is any one of: i) an X-ray imaging apparatus, ii) an MR imaging apparatus, and iii) a nuclear imaging apparatus.

13. The imaging arrangement of claim 11 or 12, wherein the X-ray imaging apparatus is a computed tomography scanner.

14. A computer program element, which, when being executed by at least one processing unit (PU), is adapted to cause the processing unit to perform the method as per any one of claims 9-10.

15. At least one computer readable medium (MEM) having stored thereon the program element, and/or having stored thereon the machine learning module as per claim 7 or 8.

FIG. 1

**FIG. 2**

FIG. 3

**FIG. 4**

**FIG. 5**

**FIG. 6**

$I_{IN}$

S710

S720

$I_{OUT}$

# FIG. 7

Europäisches
Patentamt

European
Patent Office

Office européen
des brevets

**PARTIAL EUROPEAN SEARCH REPORT**

under Rule 62a and/or 63 of the European Patent Convention.
This report shall be considered, for the purposes of
subsequent proceedings, as the European search report

Application Number

EP 20 19 4246

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHI JIANNING ET AL: "Single Low-Dose CT Image Denoising Using a Generative Adversarial Network With Modified U-Net Generator and Multi-Level Discriminator", IEEE ACCESS, IEEE, USA, vol. 8, 2 July 2020 (2020-07-02), pages 133470-133487, XP011801129, DOI: 10.1109/ACCESS.2020.3006512 [retrieved on 2020-07-28] | 1,3-6,8,9 | INV. G06T5/00 G06T5/20 G06K9/00 |
| Y | * abstract * * figures 1,2 * * sections I, III.A,III.C,III.D,IV.B * | 2 | |
| Y | PHILLIP ISOLA ET AL: "Image-to-Image Translation with Conditional Adversarial Networks", ARXIV.ORG, CORNELL UNIVERSITY LIBRARY, 201 OLIN LIBRARY CORNELL UNIVERSITY ITHACA, NY 14853, 21 November 2016 (2016-11-21), XP080733474, DOI: 10.1109/CVPR.2017.632 * abstract * * section 2.2.2 * | 2 | |

TECHNICAL FIELDS
SEARCHED (IPC)

G06T
G06K

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 12 February 2021 | Scholz, Volker |

EPO FORM 1503 03.82 (P04E07)

Europäisches
Patentamt

European
Patent Office

Office européen
des brevets

**INCOMPLETE SEARCH
SHEET C**

Application Number

EP 20 19 4246

---

Claim(s) completely searchable:
        1-6, 8, 9

Claim(s) not searched:
        7, 10-15

Reason for the limitation of the search:

In reply to the invitation to indicate the claims on which the search is
to be based, the applicant indicated independent claims 1, 8 and 9. Thus,
the search report has been drawn up on the basis of claims 1-6, 8 and 9
(Rule 62a(1) EPC).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8938110 B

- WO 2019229119 A

**Non-patent literature cited in the description**

- **T. M. MITCHELL.** Machine Learning. McGraw-Hill, 1997, 2
- **IAN GOODFELLOW et al.** *Generative Adversarial Networks,* 10 June 2014
- **P ISOLA et al.** Image-to-image translation with conditional adversarial networks. *Proceedings of the IEEE conference on computer vision and pattern recognition,* 2017, 1125-1134
- U-Net: Convolutional Networks for Biomedical Image Segmentation. **RONNEBERG et al.** Medical Image Computing and Computer-Assisted Intervention -MICCAI 2015. Lecture Notes in Computer Science, 2015, vol. 9351

- **M FREIMAN et al.** Unsupervised abnormality detection through mixed structure regularization (MSR) in deep sparse Auto-encoders. *Medical Physics,* 2019, vol. 46 (5), 2223-2231
- **I GOODFELLOW et al.** Generative adversarial nets. *NIPS'14:Proceedings of the 27th International Conference on Neural Information Processing Systems,* 2014, vol. 2, 2672-2680
- **MARJOVSKY et al.** Wasserstein GAN. *arXiv,* 2017
- **GUO-JUN QI.** Loss-sensitive generative adversarial networks on Lipschitz densities. *arXiv,* 2017